# EUROPEAN PATENT APPLICATION

(11) **EP 2 778 993 A1**
(43) Date of publication of application: **17.09.2014**
(21) Application number: 12862480.6
(22) Date of filing: 02.07.2012
(51) Int. Cl.: G06F 19/00

(54) **HEALTH DATA MANAGEMENT METHOD AND DEVICE**

(30) Priority: 29.12.2011 CN 201110453662
(71) Applicant: Huawei Technologies Co., Ltd, Shenzhen, Guangdong 518129 (CN)
(72) Inventor: XU, Zhongqing, Shenzhen Guangdong 518129 (CN); LI, Huarong, Shenzhen Guangdong 518129 (CN); WEN, Changcheng, Shenzhen Guangdong 518129 (CN)
(74) Representative: Epping - Hermann - Fischer
(86) International application number: PCT/CN2012/078025
(87) International publication number: WO 2013/097440

(57) **Abstract**

The present invention discloses a health data management method and apparatus. The health data management method includes: extracting heterogeneous health data, and generating at least one intermediate document in a key-value form, where at least one field in the heterogeneous health data corresponds to a key key in the intermediate document, and one field corresponds to one key; transforming the intermediate document into a clinical document architecture CDA document; and outputting the CDA document. The technical solution provided by the present invention can effectively increase the degree of health data sharing.

## Description

### CROSS-REFERENCE TO RELATED APPLICATION

This application claims priority to Chinese Patent Application No. 201110453662.0, filed with the Chinese Patent Office on December 29, 2011 and entitled "HEALTH DATA MANAGEMENT METHOD AND APPARATUS", which is incorporated herein by reference in its entirety.

### TECHNICAL FIELD

The present invention relates to the field of data management technologies, and in particular, to a health data management method and apparatus.

### BACKGROUND

With improvement of life, people pay more attention to physical health, leading to a stronger desire to establish an individual-centered health record warehouse.

At present, in numerous health services (POS, Point Of Service) including hospitals and disease control centers in China, most health data are constructed in customized data formats. These health data can only be used inside organizations and are difficult to be shared by external systems or checked by external users, forming an actual isolated health data island.

### SUMMARY

Embodiments of the present invention provide a health data management method and apparatus, so as to increase the degree of health data sharing.

The embodiments of the present invention provide the following technical solution:

A health data management method includes:
extracting heterogeneous health data, and generating at least one intermediate document in a key-value form, where at least one field in the heterogeneous health data corresponds to a key key in the intermediate document, and one field corresponds to one key;
transforming the intermediate document into a clinical document architecture CDA document; and
outputting the CDA document.

A health data management apparatus includes:
an extracting unit, configured to extract heterogeneous health data;
a generating unit, configured to generate at least one intermediate document in a key-value form, where at least one field in the heterogeneous health data corresponds to a key key in the intermediate document, and one field corresponds to one key;
a transforming unit, configured to transform the intermediate document into a clinical document architecture CDA document; and
an outputting unit, configured to output the CDA document.

As can be seen from the above, in the embodiments of the present invention, heterogeneous health data is extracted, the heterogeneous health data is transformed into a CDA document, and the CDA document is output, so that the heterogeneous health data format is standardized, and different health services can share their internal health data. As can be seen, the technical solution provided by the present invention effectively increases the degree of health data sharing.

### BRIEF DESCRIPTION OF DRAWINGS

To describe the technical solutions in the embodiments of the present invention or in the prior art more clearly, the following briefly introduces the accompanying drawings required for describing the embodiments or the prior art. Apparently, the accompanying drawings in the following description show some embodiments of the present invention, and a person of ordinary skill in the art may still derive other drawings from these accompanying drawings without creative efforts.
FIG. 1 is a schematic flowchart of an embodiment of a health data management method according to the present invention; and
FIG. 2 is a schematic structural diagram of an embodiment of a health data management apparatus according to the present invention.

### DESCRIPTION OF EMBODIMENTS

The following clearly and completely describes the technical solutions in the embodiments of the present invention with reference to the accompanying drawings in the embodiments of the present invention. Apparently, the described embodiments are merely a part rather than all of the embodiments of the present invention. All other embodiments obtained by a person of ordinary skill in the art based on the embodiments of the present invention without creative efforts shall fall within the protection scope of the present invention.

Embodiments of the present invention provide a health data management method and apparatus.

To make the objectives, characteristics, and advantages of the present invention more obvious and understandable, the following further clearly describes the technical solutions in the embodiments of the present invention in detail with reference to the accompanying drawings. Apparently, the described embodiments are merely a part rather than all of the embodiments of the present invention. All other embodiments obtained by a person of ordinary skill in the art based on the embodiments of the present invention without creative efforts shall fall within the protection scope of the present invention.

The clinical document architecture (CDA, Clinical Document Architecture) is a healthcare document formatting standard created by the health level 7 protocol (HL7, Health Level 7) for health information exchange. At present, a CDA document, as a most internationally popular health document standard, is accepted and adopted by more health management institutions and health services. A health record warehouse with a unified format can greatly increase the degree of health record sharing and the utilization efficiency.

In the embodiments of the present invention, heterogeneous health data is extracted, the heterogeneous health data is transformed into a CDA document, and the CDA document is output, so that the heterogeneous health data format is standardized.

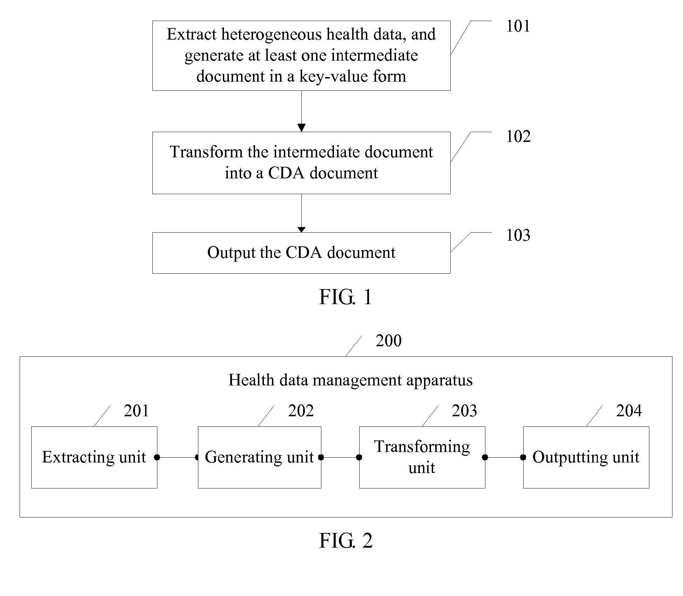

The following describes a health data management method provided by the present invention. Referring to FIG. 1, an embodiment of the health data management method in the present invention includes:

101. Extract heterogeneous health data, and generate at least one intermediate document in a key-value form.

In the embodiment of the present invention, the heterogeneous health data is extracted, and at least one intermediate document in a key-value (that is, key-value) form is generated.

The heterogeneous health data refers to health data in customized formats which is stored or generated inside medical services (such as hospitals and disease control centers).

In the embodiment of the present invention, a corresponding health data extracting template may be set in advance according to the heterogeneous health data in different formats, where a correspondence between fields and keys keys in the heterogeneous health data is defined in the health data extracting template, so that one field corresponds to one key. Before this, each key is predefined with a special meaning (such as a name, blood pressure, weight and electrocardiogram). In a practical application, the health data extracting template may include a correspondence between all fields and keys in the heterogeneous health data. Certainly, based on a practical demand, the health data extracting template may also define a correspondence between only a part of fields and keys in the heterogeneous health data, which is not limited herein.

This step is described by using an example in the following. It is assumed that the foregoing heterogeneous health data is a physical examination data table named Table A in a hospital information system of a certain hospital. The table format is defined as shown in Table 1:

**Table 1**

| Field | Name | Data Type |
|---|---|---|
| ID | Serial number of physical examination table | String |
| Patient ID | Identification card number of an examinee | String |
| Patient Name | Name of an examinee | String |
| Gender | Gender | String |
| Event Date | Date of examination | Date |
| Height | Height (centimeter) | Int |
| Weight | Weight (kilogram) | Int |
| BP | Blood pressure | String |

In a practical application, a health data physical examination template shown in table 2 is pre-defined according to the physical examination data table.

**Table 2**

| Table Name | Field Name | Corresponding Key | Default Value | Remarks |
|---|---|---|---|---|
| Table A | ID | HR01.001.01 | | Serial number of physical examination |
| | | HR01.001.02 | The People's Hospital | Physical examination organization |
| Table A | Patient ID | HR01.001.03 | | Examinee ID |
| | | HR01.001.04 | Identification Card | ID type |
| Table A | Patient Name | HR01.001.05 | | Resident name |
| Table A | Gender | HR01.001.06 | | Gender |
| Table A | Event Date | HR01.001.07 | | Start date |
| Table A | Height | HR01.001.08 | | Height |
| Table A | Weight | HR01.001.09 | | Weight |
| Table A | BP | HR01.001.10 | | Blood pressure |

It is assumed that Table A includes the records shown in Table 3:

**Table 3**

| ID | Patient ID | Patient Name | Gender | Event Date | Height | Weight | BP |
|---|---|---|---|---|---|---|---|
| 0001 | 410000111111111111 | Li San | Male | 20110623 | 175 | 63 | 120/83 |

The data in Table A is extracted according to the health data extracting template in Table 2, and one intermediate document in a key-value form is generated, where the document includes the following information:
< item key = "HR01.001.01" value = "0001"/>
<item key = "HR01.001.02" value = "The People's Hospital"/>
<item key = "HR01.001.03" value = "410000111111111111"/>
<item key = "HR01.001.04" value = "Identification card"/>
<item key = "HR01.001.05" value = "Li San"/>
<item key = "HR01.001.06" value = "Male"/>
<item key = "HR01.001.07" value = "20110623"/>
<item key = "HR01.001.08" value = "175"/>
<item key = "HR01.001.09" value = "63"/>
<item key = "HR01.001.10" value = "120/83"/>

The intermediate document is described by using the extensible markup language (XML, extensible Markup Language).

In an application scenario, one copy of heterogeneous health data usually includes more than one record. Using the foregoing physical examination data table as an example, if the physical examination data table includes more than two records, a corresponding number of intermediate documents can be generated. One intermediate document corresponds to one record. Certainly, one intermediate document corresponding to multiple records may be generated, which is not limited here. In a practical application, multiple copies of associated heterogeneous health data may also be defined in one health data extracting template. The foregoing physical examination data table is used as an example. If Table B associated with Table A exists, and an associated field is Patient ID, a correspondence between fields and keys in Table B may further be defined in Table 2. A health data management apparatus extracts heterogeneous health data with a same value of Patient ID from Table A and Table B according to the health data extracting template, and then generates an intermediate document.

### 102. Transform the foregoing intermediate document into a CDA document.

In the embodiment of the present invention, a CDA transforming template may be predefined, where the CDA transforming template is described by using the extensible stylesheet language transformation (XSLT, Extensible Stylesheet Language Transformation) language, that is, the CDA transforming template is in fact an XSLT file. The CDA transforming template defines a transforming standard for CDA documents. In step 101, after the intermediate document is generated, the health data management apparatus invokes the XSLT file to transform the intermediate document into a CDA document. Certainly, in the embodiment of the present invention, other description languages may also be adopted to define the transforming standard for CDA documents, which is not limited herein.

### 103. Output the CDA document.

The health data management apparatus outputs the CDA document obtained by transforming in step 102, so that the document can be used by other institutions or devices.

In the embodiment of the present invention, the health data management apparatus may also store the CDA document in a CDA document library, so that the document can be invoked for auditing, legal or other applications.

In the embodiment of the present invention, for the convenience of searching and analyzing data, after step 101, the intermediate document may be stored as relational health data; for the convenience of updating and management, keys in the intermediate document are mapped to preset codes, and then a relational health document obtained by mapping is stored. For example, the obtained relational health document is stored into an electronic health record (EHR, Electronic Health Record) database.

The foregoing physical examination data table, Table A, is still used as an example for description. A key mapping table shown in Table 4 may be predefined:

**Table 4**

| Key | Preset Code |
|---|---|
| HR01.001.01 | 0080000001 |
| HR01.001.02 | 0080000002 |
| HR01.001.03 | 0080000003 |
| HR01.001.04 | 0080000004 |
| HR01.001.05 | 0080000005 |
| HR01.001.06 | 0080000006 |
| HR01.001.07 | 0080000007 |
| HR01.001.08 | 0080000008 |
| HR01.001.09 | 0080000009 |
| HR01.001.10 | 0080000010 |

The keys in the generated intermediate document are mapped to preset codes by using the mapping table shown in table 4, and then relational health data shown in Table 5 may be obtained.

**Table 5**

| Key | Value |
|---|---|
| 0080000001 | 0001 |
| 0080000002 | The People's Hospital |
| 0080000003 | 410000000000000000 |
| 0080000004 | Identification Card |
| 0080000005 | Li Si |
| 0080000006 | Male |
| 0080000007 | 20110623 |
| 0080000008 | 175 |
| 0080000009 | 63 |
| 0080000010 | 120/83 |

By using the foregoing mapping manner, when key values change, it is only necessary to modify the mapping table to complete updating the relational health data without the need to modify all actual key values in the stored relational health data.

In a practical application, when a device sharing the CDA document receives a CDA document output by the health data management apparatus, on one hand, the CDA document may be stored in a local CDA document library, and at the same time, the CDA document may be parsed to extract an intermediate document in a key-value form, and the intermediate document is stored as relational health data in a local EHR database. Certainly, keys in the intermediate document may also be mapped to preset codes according to the foregoing mapping manner, and the relational health data obtained after the mapping may be stored in the local EHR database.

As can be seen from the above, in the embodiment of the present invention, heterogeneous health data is extracted, the heterogeneous health data is transformed into a CDA document, and the CDA document is output, so that the heterogeneous health data format is standardized, and different health services can share their internal health data. As can be seen, the technical solution provided by the present invention effectively increases the degree of health data sharing.

The following describes a health data management apparatus of the present invention. Referring to FIG. 2, a health data management apparatus 200 in an embodiment of the present invention includes an extracting unit 201, a generating unit 202, a transforming unit 204, and an outputting unit 204.

The extracting unit 201 is configured to extract heterogeneous health data.

The heterogeneous health data refers to health data in customized formats which is stored or generated inside health services.

In the embodiment of the present invention, a corresponding health data extracting template may be set in advance according to the heterogeneous health data in different formats, where a correspondence between fields and keys keys in the heterogeneous health data is defined in the health data extracting template, so that one field corresponds to one key. Before this, each key is predefined with a special meaning (such as a name, blood pressure, weight and electrocardiogram). In a practical application, the health data extracting template may include a correspondence between all fields and keys in the heterogeneous health data. Certainly, based on a practical demand, the health data extracting template may also define a correspondence between only a part of fields and keys in the heterogeneous health data, which is not limited herein. The extracting unit 201 extracts the heterogeneous health data according to the predefined health data extracting template.

The generating unit 202 is configured to generate at least one intermediate document in a key-value form.

In the embodiment of the present invention, the generating unit 202 generates, according to the heterogeneous health data extracted by the extracting unit 201, at least one intermediate document in a key-value (that is, key-value) form.

At least one field in the foregoing heterogeneous health data corresponds to a key in the intermediate document, and one field corresponds to one key.

The transforming unit 203 is configured to transform the intermediate document generated by the generating unit 202 into a CDA document.

In the embodiment of the present invention, a CDA transforming template may be predefined, where the CDA transforming template is described by using the XSLT language, that is, the CDA transforming template is in fact an XSLT file. The CDA transforming template defines a transforming standard for CDA documents. After the generating unit 202 generates the intermediate document, the transforming unit 203 invokes the XSLT file to transform the intermediate document into a CDA document. Certainly, in the embodiment of the present invention, other description languages may also be adopted to define the transforming standard for CDA documents, which is not limited herein.

The outputting unit 204 is configured to output the CDA document obtained by transforming by the transforming unit 203.

The outputting unit 204 outputs the CDA document obtained by transforming by the transforming unit 203, so that the document can be used by other institutions or devices.

Furthermore, the health data management apparatus further includes: a mapping unit and a first storage unit.

The mapping unit is configured to map keys in the intermediate document generated by the generating unit 202 to preset codes, where the keys and the preset codes are in one-to-one mapping; and the first storage unit is configured to store a relational health document obtained after the mapping, for example, the first storage unit may store the obtained relational health document in an EHR database.

Furthermore, the health data management apparatus further includes: a second storage unit, configured to store, in a CDA document library, the CDA document obtained by transforming by the transforming unit 203.

It should be noted that the health data management apparatus 200 in the embodiment of the present invention may be the health data management apparatus in the foregoing method embodiment, and may be configured to implement all technical solutions in the foregoing method embodiment, where functions of functional modules thereof may be specifically implemented according to the method in the foregoing method embodiment, and for the specific implementation process, reference may be made to related description in the foregoing embodiment, which is not described herein again.

As can be seen from the above, the health data management apparatus in the embodiment of the present invention extracts heterogeneous health data, transforms the heterogeneous health data into a CDA document, and outputs the CDA document, so that the heterogeneous health data format is standardized, and different health services can share their internal health data. As can be seen, the technical solution provided by the present invention effectively increases the degree of health data sharing.

It can be clearly understood by a person skilled in the art that, for the purpose of convenient and brief description, for a detailed working process of the foregoing apparatus and unit, reference may be made to a corresponding process in the foregoing method embodiments, and details are not described herein again.

The method provided by the embodiments of the present invention may be executed by a universal integrated circuit (such as a central processing unit CPU) and an application specific integrated circuit (ASIC). The apparatus and units in the embodiments of the present invention may be a base station, a universal integrated circuit (such as a central processing unit CPU), and an application specific integrated circuit (ASIC), and the like.

In the several embodiments provided in the present application, it should be understood that the disclosed apparatus and method may be implemented in other manners. For example, the described apparatus embodiment is merely exemplary. For example, the unit division is merely logic function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or not be physically separate, and parts displayed as units may or may not be physical units, may be located in one position, or may be distributed on a plurality of network units. A part or all of the units may be selected according to an actual need to achieve the objectives of the solutions of the embodiments.

In addition, functional units in the embodiments of the present invention may be integrated into one processing unit, or each of the units may exist alone physically, or two or more units are integrated into one unit. The integrated unit may be implemented in a form of hardware, or may be implemented in a form of a software functional unit.

When the integrated unit is implemented in a form of a software functional unit and sold or used as an independent product, the integrated unit may be stored in a computer-readable storage medium. Based on such an understanding, the technical solutions of the present invention essentially, or the part contributing to the prior art, or all or a part of the technical solutions may be implemented in the form of a software product. The software product is stored in a storage medium and includes several instructions for instructing a computer device (which may be a personal computer, a server, or a network device) to perform all or a part of the steps of the methods described in the embodiments of the present invention. The foregoing storage medium includes any medium that can store program code, such as a USB flash drive, a removable hard disk, a read-only memory (Read-Only Memory, ROM), a random access memory (Random Access Memory, RAM), a magnetic disk, or an optical disc.

The health data management method and apparatus provided by the present invention have been described in detail. For a person of ordinary skill in the art, variations can be made to the specific implementation manner and the application scope of the present invention based on the idea of the embodiments of the present invention. In conclusion, this specification shall not be construed as a limitation to the present invention.

## Claims

1. A health data management method, comprising:
extracting heterogeneous health data, and generating at least one intermediate document in a key-value form, wherein at least one field in the heterogeneous health data corresponds to a key key in the intermediate document, and one field corresponds to one key;
transforming the intermediate document into a clinical document architecture CDA document; and
outputting the CDA document.

2. The health data management method according to claim 1, wherein:
after the generating at least one intermediate document in a key-value form, the method further comprises:
mapping keys in the intermediate document to preset codes, wherein the keys and the preset codes are in one-to-one mapping; and
storing a relational health document obtained after the mapping.

3. The method according to claim 2, wherein:
the storing a relational health document obtained after the mapping comprises:
storing, in an electronic health record EHR database, the relational health document obtained after the mapping.

4. The health data management method according to any one of claims 1 to 3, wherein:
the transforming the intermediate document into a CDA document comprises: transforming the intermediate document by using extensible stylesheet language transformation XSLT, and generating the CDA document.

5. The method according to any one of claims 1 to 3, wherein:
after the transforming the intermediate document into a CDA document, the method further comprises:
storing the CDA document in a CDA document library.

6. A health data management apparatus, comprising:
an extracting unit, configured to extract heterogeneous health data;
a generating unit, configured to generate at least one intermediate document in a key-value form, wherein at least one field in the heterogeneous health data corresponds to a key key in the intermediate document, and one field corresponds to one key;
a transforming unit, configured to transform the intermediate document into a clinical document architecture CDA document; and
an outputting unit, configured to output the CDA document.

7. The apparatus according to claim 6, wherein:
the health data management apparatus further comprises:
a mapping unit, configured to map keys in the intermediate document generated by the generating unit to preset codes, wherein the keys and the preset codes are in one-to-one mapping; and
a first storage unit, configured to store a relational health document obtained after the mapping.

8. The apparatus according to claim 7, wherein:
the first storage unit is specifically configured to store, in an electronic health record EHR database, the relational health document obtained after the mapping.

9. The apparatus according to any one of claims 6 to 8, wherein:
the transforming unit is specifically configured to transform, by using extensible stylesheet language transformation XSLT, the intermediate document generated by the generating unit, and generate the CDA document.

10. The apparatus according to any one of claims 6 to 8, wherein:
the health data management apparatus further comprises:
a second storage unit, configured to store, in a CDA document library, the CDA document obtained by transforming by the transforming unit.
